# EUROPEAN PATENT APPLICATION

(11) **EP 4 570 361 A1**
(43) Date of publication of application: **18.06.2025**
(21) Application number: 23215656.2
(22) Date of filing: 11.12.2023
(51) Int. Cl.: B01D 61/18, B01D 61/20, B01D 61/22, C12M 3/06, C12M 1/00, C12N 15/00

(54) **DEVICE ASSEMBLY AND METHOD FOR A PRODUCTION-SCALE TANGENTIAL FLOW FILTRATION**

(71) Applicant: Sartorius Stedim Biotech GmbH, 37079 Göttingen (DE)
(72) Inventor: Hack, Alexander, 37079 Göttingen (DE); Lobedann, Martin, 37079 Göttingen (DE)
(74) Representative: Prinz & Partner mbB

(57) **Abstract**

A device assembly (10) for performing a production scale tangential flow filtration of a feed, preferably in a batch feed and bleed process, comprises a first loop (12) including a first tubing (20), a feed tank (22) and actuators and sensors adapted to the first tubing (20), and a second loop (14) including second tubing (28) and a filter (28), preferably a hollow fiber filter. The first loop (12) connects to the second loop (14) at a first connection point (16) upstream, or alternatively downstream, of the filter (28), and the second loop (14) connects to the first loop (12) at a second connection point (18) downstream, or alternatively upstream, of the filter (28), such that the first loop (12) supplies the feed from the feed tank (22) to the second loop (14) at the first connection point (16), or alternatively at the second connection point (18), and the second loop (14) supplies retentate to the first loop (12) at the second connection point (18) from where a part of the retentate is returned to the feed tank (22), or alternatively at the first connection point (14). At least all wetted parts of the device assembly (10) are single-use parts and the inner diameter of the first tubing (20) is smaller than the inner diameter of the second tubing (32).

## Description

The invention relates to a device assembly for performing production-scale tangential flow filtration of a feed. The invention further relates to a method of performing production-scale tangential flow filtration of a feed.

Tangential flow filtration (TFF) is a common processing step in concentration and diafiltration operations in the downstream processing of pharmaceutical products.

In particular, TFF may be used to harvest and clarify plasmid DNA (pDNA) which is a critical starting point for many genetic engineering pursuits, including the development of recombining proteins, viral vectors, and advanced bio therapeutics. Purified pDNA can be used as a raw material in processes such as the production of adeno-associated viruses (AAV) and lentiviral vectors, in vitro transcription (IVT) reactions during mRNA production, and direct gene transfer. Consequently, tangential flow filtration is also used for mRNA production. To use TFF for pDNA or mRNA, membranes with a molecular weight cut-off (MWCO) of 500 to 750 kDa corresponding to pore sizes of 0.1 µm to 1 mm are required.

TFF can also be used for human and animal vaccine production as well as lipid nanoparticle (LNP) purification with an MWCO between 10 kDa and 750 kDa, and a pore size between 0.1 and 10 µm.

Further, TFF can also be used for lentiviral vector (LV) filtration which requires a large bioreactor, e. g. with a volume of 500 L. LV-filtration is limited by the downstream process (DSP) speed such that it is often better to have smaller batches.

Using pre-sanitized, gamma irradiated, or pre-sterilized disposable TFF membranes as well as single-use sensors, pumps, and tubes, leads to a decrease in the resources required to complete a process. Resources in that sense refer to labor, buffer and water usage, as well as costs.

A typical reusable TFF system will involve some 10 major process steps, such as setup, clean-in-place (CIP), flush, perform normalized water permeability (NWP) / membrane integrity tests, equilibrate with sample buffer, process the product, CIP, flush, NWP, and storage. By using a single-use TFF device assembly, the number of process steps can be significantly reduced.

Over the last decade, the bioprocessing industry has recognized that single-use equipment can provide significant savings in time, labor, and capital. As a scalable technology, the flexibility of single-use disposables has increased production capacity, eliminated CIP cleaning and validation, sterilize-in-place (SIP), and has reduced the use of caustic chemicals and water for injection. Additionally, the use of single-use equipment can reduce the risk of cross contamination between different batches.

This philosophy also applies to TFF. However, many of the devices needed to scale up single-use TFF devices assemblies from bench-top scale to production scale do not currently exist. For example, there are no conductivity sensors, valves, or tubing with validated end fittings at the desired size, especially for single-use systems with a desired tubing diameter of 2" (5.08 cm) or more. There is also no retentate vessel available with the required port size.

There have been multiple attempts to scale up the bench-top single-use equipment. However, they all come with disadvantages that need to be avoided. The most obvious approach is to use larger filters in order to scale up. However, such a scale-up involves using larger pumps, tubing, valves, sensors, and mixer ports. As already mentioned, none of these parts are currently available in the desired larger sizes. Furthermore, there are significant challenges preventing the creation of these missing parts. Using the currently available smaller pumps, tubing, sensors etc. in order to employ a larger filter is not possible, since they cannot provide the required flow rate.

The largest, currently available valves for single-use tubing have an inner diameter of 1" (2.54 cm). However, an inner diameter larger than 1" is required for efficient production. In particular, tubing with an inner diameter of 2" or larger is needed in order to provide the desired flow rate of at least 180 L/min. Having such a high flow rate in a 1" pipe would result in turbulences and product loss. While tubing is available up to an inner diameter of 2" (5.08 cm), these tubes are not available with hose barbs on their ends to allow a connection to other pipes. In addition, fixture devices such as ear clamps are inadequate to securely fix the flexible hose onto a rigid hose barb. Moreover, further problems may occur when the tube has a significant length since a large pressure drop is generated across the tube length. Consequently, the tube can be sucked shut (collapses) on the suction side of the pump. The main reason for this is a reduced ratio of wall thickness to the inner diameter as the tube size increases. Neither sensors nor mixer ports are available for inner diameters larger than 1", either.

An alternative approach has been to use filters one at a time, i. e. smaller filters which require a lower flow rate. This results in a longer processing time since the permeate flow rate is limited to the size of the smaller filter. Furthermore, the volume and/or time that each of the filters is operated is not equal, meaning the first filters will foul faster than the later filters. This method is currently used on horizontal flow - vertical flow (HF VF) filtration.

Multiple smaller filters can be used in a series configuration. However, the pressure at each filter entrance will differ, resulting in preferential flow to the earlier filters.

Similarly, a configuration using multiple filters in parallel exhibits an uneven flow distribution

The most common way to scale up single-use TFF is to use multichannel approaches, wherein several 1" setups are connected to a common feed tank. They exist and work; however, they have a large footprint and involve high costs.

Therefore, it is an objective of the invention to provide a device assembly for large volumes and single use without significantly increasing the cost and footprint compared to current single-use TFF devices.

This objective is solved by a device assembly according to claim 1. The device assembly for performing a production scale tangential flow filtration of a feed, preferably in a batch feed and bleed process, comprises a first loop including a first tubing, a feed tank and actuators and sensors adapted to the first tubing. The device assembly further comprises a second loop including second tubing and a filter, preferably a hollow fiber filter. The first loop connects to the second loop at a first connection point upstream of the filter, and the second loop connects to the first loop at a second connection point downstream of the filter, such that the first loop supplies the feed from the feed tank to the second loop at the first connection point, and the second loop supplies retentate to the first loop at the second connection point from where a part of the retentate, called "the bleed", is returned to the feed tank. At least all wetted parts of the device assembly are single-use parts, and the inner diameter of the first tubing is smaller than the inner diameter of the second tubing.

According to an alternative solution of the invention, the first loop connects to the second loop at a first connection point downstream of the filter and the second loop connects to the first loop at a second connection point upstream of the filter such that the first loop supplies the feed from the feed tank to the second loop at the second connection point and the second loop supplies retentate to the first loop at the first connection point. For the sake of convenience, the following description mainly relates to the alternative described further above (first connection point upstream of the filter, second connection point downstream of the filter), but shall apply accordingly also to the other alternative.

The invention is based on the finding that the limitations on the scale can be eliminated by separating the flow path into two loops, wherein only components requiring a high flow rate are placed into a second loop having a larger diameter than a first loop, which comprises the rest of the components. In particular the filter requires a high flow rate in order to generate the necessary sweeping action across the face of the hollow fiber. To generate the sweeping action, a pump is needed in the second loop, and in order to monitor the flow rate, a flow meter may be placed in the second loop. The size of all other components, such as sensors measuring pressure, conductivity, UV, pH, temperature, as well as sample ports, valves and the connection to the feed tank, does not need to be increased such that they remain in a loop with a smaller inner diameter of the tubing.

Alternatively, instead of a hollow fiber filter, which is preferred, a filter cassette can be used as well.

According to a preferred embodiment, the second tubing has a standard diameter of 2" (5.08 cm) or more. Therefore, the second tubing is scaled up compared to the standard tubing currently used in single-use TFF applications.

In order to minimize the costs, the first tubing has a standard inner diameter of 1" (2.54 cm) or less. This corresponds to standard single-use sizing, meaning it is already available and does not increase the costs of the filtration set-up.

The exact size of the first loop will depend on the required flow rate for the filter. In particular, the size of the tubing for the first loop is chosen such that the pipe velocity does not exceed 2 m/s or 3600 l/h. The limit of 3600 l/h is due to the pump used in the setup in order to reduce shear and pressure.

According to a preferred embodiment, the second tubing includes rigid piping while the first tubing preferably includes flexible hoses. In particular, the second loop comprises predominantly rigid piping opposed to the preferably flexible tubing of the first loop. By having the second tubing in form of (predominantly) rigid pipes, potential collapse of the tubing on the side of the suction pump can be avoided. The term "rigid piping" refers to a pipe that is molded from materials such as polypropylene or polyvinylidene (di)fluoride (PVDF), whereas the term "flexible tubing" or "flexible hoses" refers to hoses made from materials such as platinum cured silicone.

Further, the device assembly comprises control means configured to control the flow rate and the pressure in the first loop and in the second loop, especially the transmembrane pressure (TMP) across the filter. These control means are of utmost importance since the filtration process depends on the TMP and flow rate. The TMP is the driving force for liquid transports to the filter membrane such that a TMP which is too low results in no filtration whereas a TMP which is too high leads to damages of the filter membrane.

In particular, the TMP is defined by the feed pressure before and after the feed is pressurized, and the permeate pressure. Due to the two loop design of the device assembly, the pressurized feed is approximately equal to the TMP.

According to a preferred embodiment, the control means are configured such that the flow rate and the pressure in the entire second loop are high, whereas the flow rate and the pressure in the tubing directly leading to and away from the feed tank in the first loop, i. e. the tubes directly connected to the feed tank, are low. In other words, the flow rate in the first loop is lower than the flow rate in the second loop. The high pressure needed to provide the TMP is generated in the first loop by a feed pump at a lower flow rate, thus reducing the power consumption. Moreover, since not the whole system needs to be pressurized the overall energy requirement is reduced.

In particular, compared to the device assembly according to the invention, traditional TFF uses about ten times more energy since the complete high flow rate feed stream needs to be pressurized. On the other hand, according to the invention, only the small feed stream connecting to the second loop needs to be pressurized, whereas the high flow rate circulation in the second loop stays at a high pressure.

Furthermore, a protein will pass from the high pressure zone to the low pressure zone ten times less frequently in the device assembly according to the invention compared to traditional TFF. Therefore, the stress to the target protein is reduced.

With respect to the relative terms "high" and "low" used herein, it is to be noted that a "high" pressure is always greater than a pressure indicated as "low", irrespective of where in the device assembly the pressure is considered. The same holds for the flow rate, i. e. a "high" flow rate is always greater than a "low" flow rate, and for other parameters.

Furthermore, in traditional TFF the end of the filtration process can easily be predicted in terms of concentration factors, which provide predictable end protein concentrations in the retentate vessel. However, in the batch feed and bleed concept having two different loops, i. e. a first loop and a second loop, the two loops lead to differing protein concentrations in the two loops. For optimal operation conditions, the difference between the two loops needs to be minimized. This is achieved by increasing the flow rate of the first loop, thereby increasing the mass transfer between the two loops.

In order to predict an end protein concentration, the concentration in both loops needs to be considered, making the whole calculation more complicated. This is particularly important when a sample is taken midway through the process, as differences in the sample concentration compared to the expected concentration may be attributed to the concentration gradient. It is possible to stop the permeate pump for a short amount of time, preferably not more than one minute, such that the two loops can homogenize and allow a representative sample to be taken. That way complicated calculations can be avoided.

Preferably, the control means include a feed pump in the first loop downstream of the feed tank configured to supply the feed to the second loop with a low flow rate and a high pressure, a first circulation pump, preferably a centrifugal pump, in the second loop upstream of the filter, and a retentate control valve in the first loop upstream of the feed tank. The transmembrane pressure is mainly controlled jointly by the feed pump in the first loop and the retentate control valve. The control valve is configured to provide a controlled back pressure, meaning the control valve in the first loop can help control the pressure in the second loop and, thus, the transmembrane pressure. Since the control valve is located in the small loop, i. e. the first loop, it does not need to be scaled up, which results in a simplified construction and an increased lifetime of the control valve. The first circulation pump is used to compensate the pressure loss caused by friction in the second loop.

According to an alternative second concept of the invention, the control means include a feed pump in the first loop downstream of the feed tank configured to supply the feed to the second loop with the high flow rate and the high pressure, a first circulation pump, preferably a centrifugal pump, in the second loop upstream of the filter, and a bleed pump in the first loop upstream of the feed tank. The transmembrane pressure is mainly controlled jointly by the feed pump and the bleed pump. In this configuration, the bleed pump controls the backpressure and consequently the transmembrane pressure. In particular, the bleed pump simultaneously controls the transmembrane pressure and the permeate flow by controlling the flow rate difference between the feed and bleed pumps, i. e. the feed and circulation pumps.

According to a further alternative third configuration, the control means are configured such that in the second loop, the flow rate and the pressure are high upstream and downstream of the filter, the flow rate is high and the pressure is low at the first and second connection points, and in the whole first loop, the flow rate and the pressure are low. In this configuration, the energy requirement is reduced since not the whole first and second loops need to be pressurized.

According to an embodiment of the third configuration, the control means include a feed pump in the first loop downstream of the feed tank configured to supply the feed to the second loop with the flow rate and the low pressure, a first centrifugal circulation pump and a second centrifugal pump in the second loop upstream and downstream of the filter respectively. The transmembrane pressure is mainly controlled jointly by the first and second centrifugal circulation pumps, the first and second centrifugal circulation pumps being configured to being capable of establishing a first flow direction over the filter and an alternative (opposite) second flow direction over the filter. This means that this embodiment allows for a so-called alternative tangential flow (ATF) operation. Besides the possibility of operating the device assembly in an ATF mode, it can still be operated in the standard feed-and-bleed mode.

Since ATF is considered as an optimized version of TFF, the possibility of operating the embodiment in an ATF mode has multiple advantages. For one, the perfusion process is designed to minimize filter fouling such that the filter can be used longer since its lifetime is increased. The retentate being moved back and forth across the membrane cleans the filter.

In this configuration centrifugal pumps need to be used since they are not positive displacement pumps. That means that fluid can easily be pushed through the pump, even in the reverse direction, i. e. the direction opposite to the pumping direction. If the centrifugal pump upstream of the filter operates at high revolutions per minute (rpm) in order to achieve the desired flow rate and the centrifugal pump downstream of the filter pumps at lower rpm in the opposite direction, the second centrifugal pump provides enough dynamic back pressure to control the transmembrane pressure while still allowing the first centrifugal pump to provide the dominant flow direction and flow rate control. However, compared to the previous embodiments, this embodiment has a slightly higher energy consumption as the second recirculation pump requires energy in order to generate the dynamic back pressure.

Furthermore, centrifugal pumps are available as single-use equipment and are able to provide the necessary high flow rates required to operate a larger filter. Consequently, the centrifugal pump method eliminates the problem of single-use control valves, which are not available at this larger size. The function of the centrifugal pumps can be switched such that the second centrifugal pump provides the flow direction and flow rate while the first centrifugal pump operates at lower rpm setting and the flow direction changes from anticlockwise to clockwise. During the process, this switching can occur every few minutes in order to remove the fouling layers from the filter which in turn boosts the permeate flux.

In a further alternative configuration, the control means are configured such that in the whole second loop the flow rate and the pressure are high, whereas in the first loop, the flow rate and the pressure in the tubing directly leading to and away from the feed tank, i. e. the tubes directly connected to the feed tank, are low. Again, the energy requirement is reduced since only the second loop needs to be pressurized (e. g. by a feed pump provided in the first loop).

According to a preferred embodiment of this configuration, the control means include a feed pump in the first loop downstream of the feed tank configured to supply the feed to the second loop with a low flow rate and a high pressure, a first circulation pump and a first circulation on/off valve connected in parallel upstream of the filter in a second loop, a second circulation pump and a second circulation on/off valve connected in parallel downstream of the filter in the second loop, and a retentate control valve in the first loop upstream of the feed tank. The transmembrane pressure is mainly controlled jointly by the feed pump and the retentate control valve. The first and second circulation pumps and the first and second circulation on/off valves are configured to establish a first flow direction over the filter and an alternative (opposite) second flow direction over the filter. Consequently, this embodiment also allows for ATF functionality while also being operable in the feed and bleed mode.

According to a further configuration, the control means include a feed pump in the first loop downstream of the feed tank configured to supply the feed to the second loop with a low flow rate and a high pressure, a first circulation pump in the second loop upstream of the filter, one or more circulation on/off valves in different lines of the second loop, and a retentate control valve in the first loop upstream of the feed tank. The transmembrane pressure is mainly controlled jointly by the feed pump and the retentate control valves. The circulation on/off valves are configured to establish a first flow path in which the first circulation pump provides a first flow direction with the filter, and an alternative second flow path in which the circulation pump provides a second flow direction with the filter. Again, this embodiment allows for the operation in ATF mode as well as in feed and bleed mode.

According to embodiment first variant, the second loop includes a plurality of filters connected in parallel. Alternatively, according to a second variant, the second loop includes a plurality of filters connected in series. These arrangements allow to move a larger volume without further increasing the filter size.

According to a preferred embodiment, the device assembly further comprises a permeate pump, preferably a peristaltic pump, configured to control the flow rate and the pressure of the permeate. By including a permeate pump, the liquid flow can be controlled better.

It is to be noted that the permeate flow does not necessarily require a permeate pump since the TMP also controls the permeate flow rate. A higher TMP means that more of the feed is pushed through the filter at a higher pressure such that the filtration rate is increased, meaning more of the feed is filtered in a short amount of time such that the permeate flow rate increases. Consequently, a lower TMP results in a lower permeate flow rate.

The use of a peristaltic pump is particularly advantageous since it is a positive displacement pump comprising a flexible tube containing the fluid. Therefore, the pump itself can be reused in multiple different processes and only the tube inside the pump needs to be exchanged. This reduces the cost associated with the permeate pump.

In an enhanced embodiment of the invention, the device assembly further comprises a UV measurement or UV-Vis spectroscopy setup in the permeate stream configured to detect breakthrough of the filter and/or a UV measurement or UV-Vis spectroscopy setup in the retentate stream configured to monitor an analyte. In connection with a real-time analysis a fast reaction is possible.

Preferably, the pumps and valves of the device assembly are controlled by a central process control unit or a SCADA system. This simplifies the operation process, saves labor costs and improves the monitoring process.

According to a preferred embodiment, the central process control unit or the SCADA system is configured to control the concentration gradient between the first loop and the second loop, preferably by adjusting a ratio of feed stream to bleed stream being directed from and redirected to the feed tank, respectively. In other words, the central process control unit or the SCADA system is configured to prevent an overconcentration of the feed in the second loop. This is particularly important since the inventive concept will result in different concentrations in the second loop and in the bleed line on the one hand and the feed line and the feed tank on the other hand.

The relationship between the concentration in the first and second loop is linked to the ratio of the bleed flow rate in the feed line and the permeate flow rate. An equilibrium between the two loops exists; however, it is not a steady state equilibrium since the concentration in the feed tank is continuously increasing. When filtration resistance is building up, the concentration factor in the second loop, which can be considered as a dimension of freedom, can be reduced to avoid overpressure. Since concentration is supposed to increase over time, reduction of the concentration factor results in slower concentration increase, and thus in lower permeate flow. For example, If the concentration ratio of bleed to permeate is 10:1, then the concentration in the second loop is 10% higher; if the concentration ratio of bleed to permeate is 5:1, then the concentration in the second loop is 20% higher; if the concentration ratio of bleed to permeate is 3:1, then the concentration in the second loop is 33% higher.

The invention also provides a method of performing production-scale tangential flow filtration of a feed, preferably in a batch feed and bleed process, wherein the method makes use of the device assembly according to the invention and comprises the following steps: Either the first loop supplies feed from the feed tank to the second loop at the first connection point, and the second loop supplies retentate to the first loop at the second connection point from where a part of the retentate is returned to the feed tank; or the first loop supplies feed from the feed tank to the second loop at the second connection point, and the second loop supplies retentate to the first loop at the first connection point from where a part of the retentate is returned to the feed tank.

Further features and advantages of the invention will become apparent from the following description and from the accompanying drawings to which reference is made. In the schematic drawings,
- Figure 1 shows a device assembly according to a first embodiment;
- Figure 2 shows a device assembly according to a second embodiment;
- Figure 3 shows a third embodiment of a device assembly;
- Figure 4 shows a device assembly according to a fourth embodiment;
- Figure 5a shows a device assembly according to a fifth embodiment;
- Figure 5b shows the fifth embodiment in the regular feed and bleed mode;
- Figure 5c shows the fifth embodiment in the ATF mode;
- Figure 6a shows a device assembly according to a sixth embodiment:
- Figure 6b shows the sixth embodiment in the regular feed and bleed mode;
- Figure 6c shows the sixth embodiment in the ATF mode;
- Figure 7 shows a possible configuration of the fourth embodiment; and
- Figure 8 shows a possible embodiment of the first embodiment.

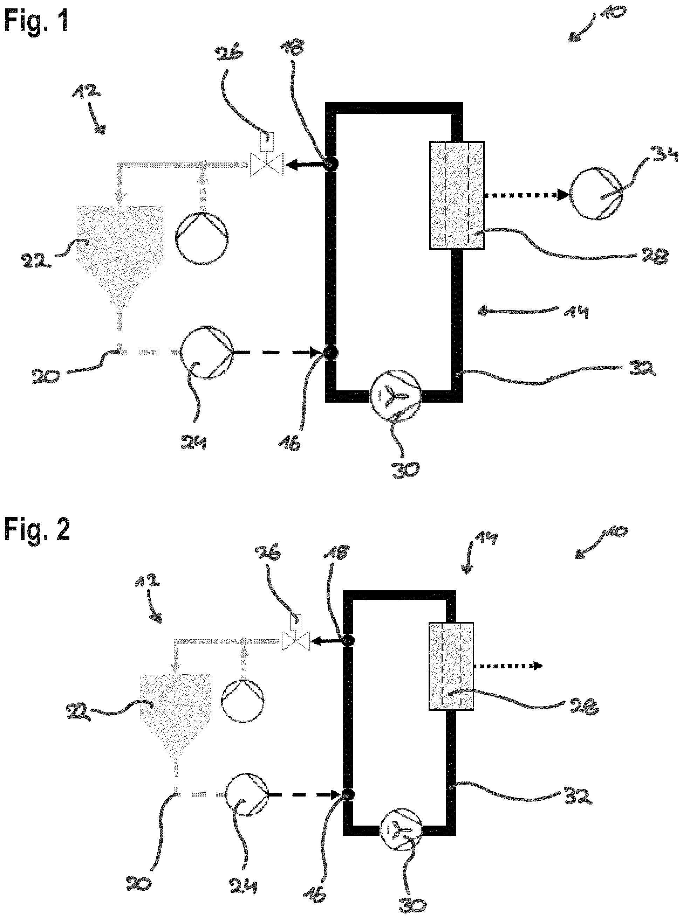

Figure 1 shows a schematic drawing of a device assembly 10 for performing production-scale tangential flow filtration of a feed, comprising a first loop 12 and a second loop 14. The two loops 12, 14 are connected to each other at a first connection point 16 and a second connection point 18.

The first loop 12 comprises a first tubing 20, preferably including flexible hoses, having a standard inner diameter of 1" (2.54 cm). The first loop 12 further comprises actuators and sensors, which are not shown in the Figures, a feed pump 24 as well as a control valve 26. The feed pump 24 is located downstream of the feed tank 22 and upstream of the first connection point 16 while the control valve 26 is located downstream of the second connection point 18 and upstream of the feed tank 22.

The second loop 14 comprises a filter 28, in particular, a hollow fiber filter, and a circulation pump 30 upstream of the filter 28.

The second loop 14 also comprises a second tubing 32, which has a standard inner diameter of 2" (5.08 cm), or more. Preferably, the second tubing 32 includes rigid piping.

All wetted parts of the device assembly 10 are single-use parts, so that they can be disposed of, preferably still assembled together, when the filtration process is completed.

The circulation pump 30 preferably is a centrifugal pump. Alternatively, the circulation pump 30 can also be a diaphragm or piston pump. However, at the desired size of the second tubing 32, centrifugal pumps are available and relatively cheap, in particular in view of the single-use aspect. The first connection point 16 is located downstream of the feed pump 24 and upstream of the circulation pump 30, while the second connection point 18 is located downstream of the filter 28 and upstream of the control valve 26.

In the illustration of this and all other embodiments, a low flow rate is indicated by a thin line, a high flow rate is indicated by a thick line, a low pressure is indicated by a bright (light grey) line, a high pressure is indicated by a dark (black) line, a high product concentration is indicated by a solid line, a low product concentration is indicated by a dashed line, and a zero product concentration is indicated by a dotted line.

From the feed tank 22 the feed is supplied to the second loop 14 at a low flow rate. When the feed first leaves the feed tank 22, it has a low pressure. After passing through the feed pump 24, the pressure of the feed is increased, such that it is now provided to the second loop 14 via the second connection point 18 at a high pressure.

In contrast to the first loop 12, the second loop 14 has a high flow rate, as well as a high pressure. The circulation pump 30 in the second loop 14 ensures that the cross-flow rate needed for the filter 28 is achieved. This means that the circulation pump 30 adjusts the flow rate according to the requirements of the filter 28 and compensates for pressure losses caused by friction in the filter 28 as well as in the second tubing 32. However, the overall flow rate and pressure in the second loop 14 are still high.

After passing through the circulation pump 30, the feed is directed to flow through the filter 28. In the filter 28, the feed passes parallel to a membrane and due to a pressure difference applied to the filter 28, constituents of the feed that are small enough to travel through the structure of the membrane pass through the membrane into a separate stream, namely the permeate.

The permeate stream has a low flow rate and a high pressure and is controlled by a permeate pump 34. The permeate pump 34 is preferably a peristaltic pump.

The constituents, which are too large to pass through the membrane of the filter 28, exit the filter 28 as the retentate stream, which flows from the filter 28 to the second connection point 18. At the second connection point 18 the retentate stream is split up such that part of the stream continues to circulate in the second loop 14 while the other part of the retentate stream is supplied back into the first loop 12. The control valve 26 regulates the amount of the retentate stream supplied to the first loop 12. In particular, the control valve 26 is configured such that it reduces the pressure of the retentate passing through while maintaining the flow rate.

The combination of the feed pump 24 and the control valve 26 is used to control the transmembrane pressure (TMP). By adjusting the amount of retentate that is allowed to pass through the control valve 26 and by adjusting the revolutions per minute (rpm) of the feed pump 24, the TMP required by the filter 28 can be achieved and adjusted throughout the process. The main purpose of the circulation pump 30 is to generate a large tangential flow regime to keep the fouling layer on the membrane as thin as possible, and thus to keep the TMP as low as possible.

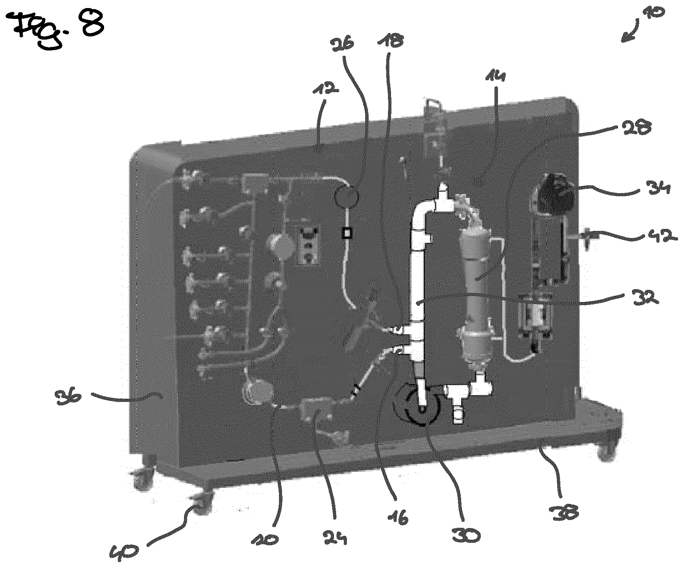

A possible embodiment of the device assembly 10 schematically shown in Figure 1 can be seen in Figure 8. Next to the two loops 12 and 14, which are mounted onto a stand 36 on top of a platform 38 comprising wheels 40, the device assembly 10 also comprises a UV-Vis spectroscopy setup 42 which is mounted onto the right side of the stand 36.

The UV-Vis spectroscopy setup 42 can be used for detecting breakthrough of the filter 28 and/or for monitoring an analyte depending on whether the UV-Vis spectroscopy setup 42 is located in the permeate stream and/or the retentate stream. In the configuration shown in Figure 8, the UV-Vis spectroscopy setup 42 is used for detecting breakthrough of the filter 28 because it is located in the permeate stream.

Instead of a UV-Vis spectroscopy setup 42 a UV measurement setup measuring at a single or multiple wavelengths can be used.

Since the control valve 26 is located in the first loop 12, it is not subjected to the same flow rates as the filter 28, which is situated in the second loop 14. That means, even though the control valve 26 together with the feed pump 24 controls the TMP, it is not subjected to the high flow rates of the second loop 14. In particular, since he volumetric flow through the control valve 26 in the first loop is reduced, the control valve 26 may have a smaller building size and increased lifetime. Further, the control valve 26 only needs to be designed to fit standard inner diameters of one inch or less, since those are the inner diameters corresponding to the first tubing 20.

A second configuration of the device assembly 10 is shown in Figure 2. The difference to the device assembly 10 shown in Figure 1 is that the device assembly 10 shown in Figure 2 does not comprise a permeate pump 34. Instead, the permeate outlet is always open, allowing the permeate stream to leave the filter 28 consistently at ambient pressure.

Therefore, the pressure and flow rate are solely controlled by the control valve 26, which generates a backpressure in combination with the feed pump 24.

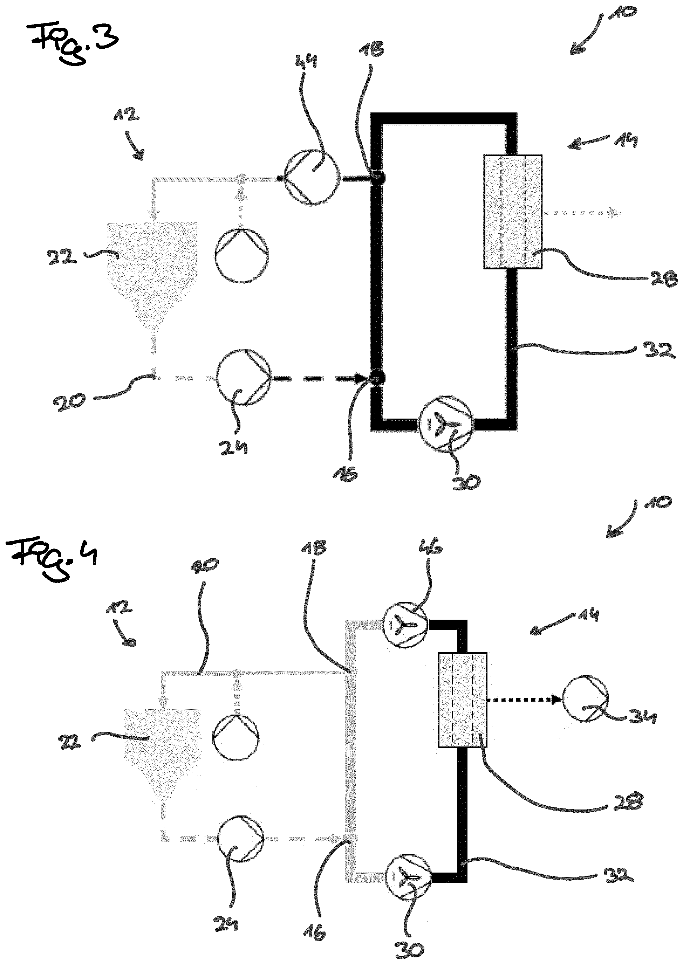

In Figure 3, a further setup of the device assembly 10 is shown, wherein a bleed pump 44 replaces the control valve 26. The bleed pump 44 is preferably a peristaltic pump, which sets the speed of the retentate stream, thereby regulating the transmembrane pressure and consequently the permeate flow rate together with the feed pump 24.

In Figures 1 to 3, the control means are provided by the feed pump 24 and the control valve 26 or the bleed pump 44, all located in the first loop 12. In the setup shown in Figure 4, the control means are solely located in the second loop 14. That means, while the feed tank 22 and the feed pump 24 as well as sensors and actuators are still located in the first loop 12, the first loop 12 does not comprise a control valve 26 or a bleed pump 44. Instead, the second loop 14 comprises the first circulation pump 30, wherein the circulation pump 30 is a centrifugal pump, as well as a second centrifugal pump 46. Since centrifugal pumps are no positive displacement pumps, the retentate can be pushed through the first or second centrifugal pump 30, 46 depending on the flow direction, even against the respective pumping direction. Note that the second centrifugal pump 46 is located downstream of the filter 28, but upstream of the second connection point 18. In this embodiment, the flow rate is a high flow rate in the whole second loop 14, but only between the two centrifugal pumps 30, 46 in the second loop 14 is the highpressure area.

When the first centrifugal circulation pump 30 is operated at higher revolutions per minute than the oppositely operated second centrifugal pump 46, the feed flows through the first centrifugal pump 30, the filter 28 and the second centrifugal pump 46 in a counterclockwise direction. However, due to comprising two centrifugal pumps 30, 46, the device assembly 10 can be operated in an alternative tangential flow (ATF) mode, wherein the second centrifugal pump 46 is operated at higher revolutions per minute than the oppositely operated centrifugal circulation pump 30. This results in a clockwise flow of the feed through the filter 28.

The switching of the flow direction from counterclockwise to clockwise can occur every few minutes during the process and helps in extending the lifetime of the filter 28, since in the ATF mode fouling layers are removed from the filter membrane more effectively than in simple TFF mode.

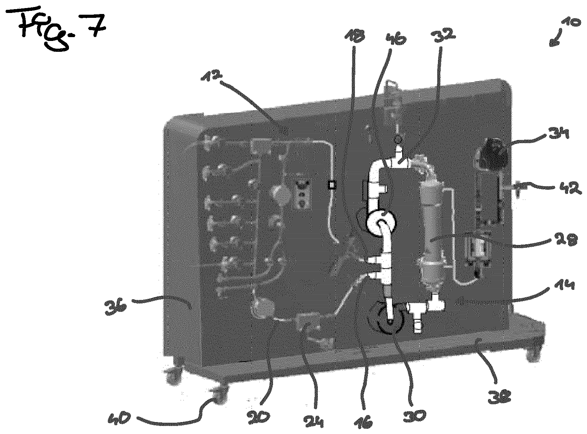

A possible configuration for the device assembly 10 of the fourth embodiment is shown in Figure 7. Similarly to the device assembly 10 shown in Figure 8, the device assembly 10 shown in Figure 7 also comprises a stand 36, a platform 38 and wheels 40. The device assembly 10 further comprises a UV measurement or UV-Vis spectroscopy setup 42 for detecting breakthrough in the permeate stream.

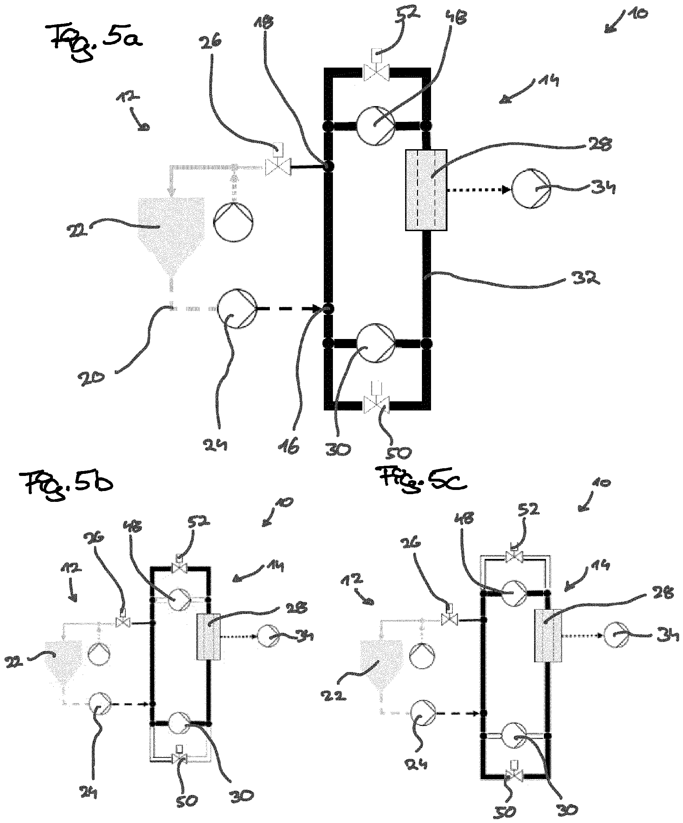

Figure 5a shows another embodiment of the device assembly 10 capable of operating in an ATF mode. However, here the control means are not provided by two centrifugal pumps 30, 46 as in Figure 4. Instead, the control means are provided by an assembly of pumps and valves. In particular, this assembly includes the circulation pump 30 which in this case is a first diaphragm pump, a second diaphragm pump 48, a first circulation on/off valve 50 connected parallel to the circulation pump 30 upstream of the filter 28 and a second circulation on/off valve 52 connected in parallel to the second diaphragm pump 48 downstream of the filter 28. The control means further comprises the control valve 26 situated in the first loop 12.

In this device assembly 10 the entire second loop 14 has a high flow rate as well as a high pressure.

By using a combination of diaphragm pumps 30, 48 and circulation on/off valves 50, 52, the need for centrifugal pumps is eliminated while still allowing the flow direction to alternate. In Figure 5b, a counterclockwise flow of the feed through the filter 28 is shown, while Figure 5c shows a clockwise flow of the feed through the filter 28.

In a counterclockwise flow, the first circulation on/off valve 50 is closed such that the circulation pump 30 pumps the feed into the filter 28. The retentate leaving the filter 28 then flows through the second circulation on/off valve 52, which in this filtration mode is open, and the second diaphragm pump 48 is turned off.

For a clockwise flow of the feed through the filter 28, the settings are reversed. This means, the second diaphragm pump 48 is on while the circulation pump 30 is turned off and the second circulation on/off valve 52 is closed while the first circulation on/off valve 50 is open.

In either of the operation modes, the feed is pumped through the filter 28 resulting in the separation of the permeate and the retentate stream. The retentate continues the circulation in the second loop 14, while the permeate leaves the filter 28, the permeate stream being controlled by the permeate pump 34.

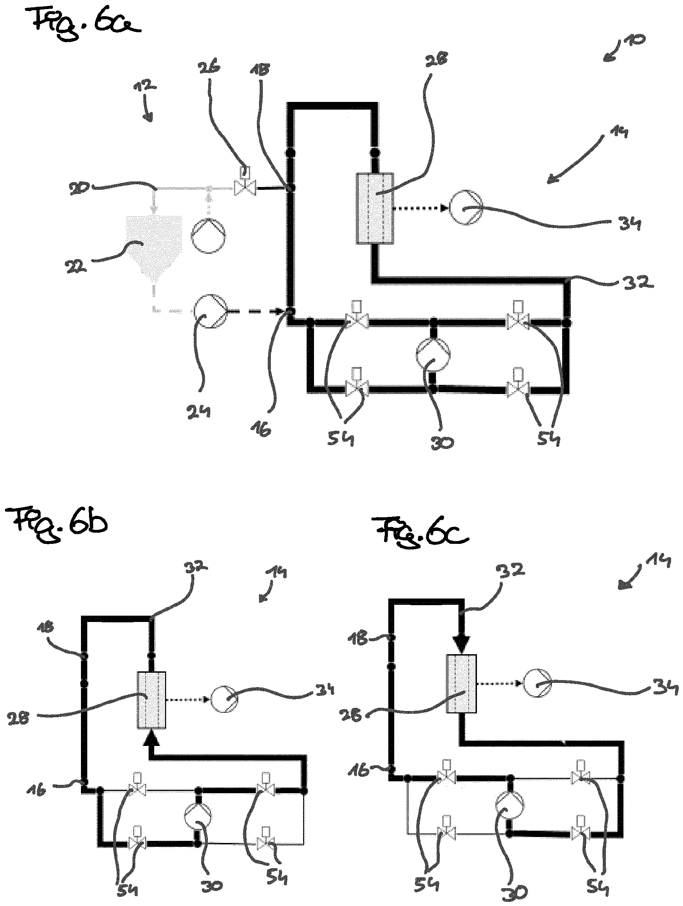

In a sixth embodiment of the device assembly 10 shown in Figure 6a, the control means are comprised by the feed pump 24 and several circulation on/off valves 54. Two of the circulation on/off valves 54 are located downstream of the filter 28, while further two circulation on/off valves 54 and the circulation pump 30 are located upstream of the filter 28.

Since the circulation pump 30 is a positive displacement pump, the flow direction depends on which of the circulation on/off valves 54 are switched on and which are switched off. The circulation on/off valves 54 determine the flow path and the flow direction of the feed and the retentate while the circulation pump 30 determines the flow rate. The counterclockwise flow is shown in Figure 6b, while the clockwise flow is shown in Figure 6c.

While some preferred embodiments have been described above, it is possible to combine certain features of these embodiments in a suitable manner.

### List of Reference Signs

- 10: device assembly
- 12: first loop
- 14: second loop
- 16: first connection point
- 18: second connection point
- 20: first tubing
- 22: feed tank
- 24: feed pump
- 26: control valve
- 28: filter
- 30: circulation pump
- 32: second tubing
- 34: permeate pump
- 36: stand
- 38: platform
- 42: UV-Vis spectroscopy setup
- 44: bleed pump
- 46: centrifugal pump
- 48: diaphragm pump
- 50: circulation on/off valve
- 52: circulation on/off valve
- 54: circulation on/off valves
- 56: connectors
- 58: flow meter

## Claims

1. Device assembly (10) for performing a production-scale tangential flow filtration of a feed, preferably in a batch feed and bleed process, wherein the device assembly (10) comprises
a first loop (12) including a first tubing (20), a feed tank (22), and actuators and sensors adapted to the first tubing (20); and
a second loop (14) including second tubing (32) and a filter (28), preferably a hollow fiber filter;
wherein the first loop (12) connects to the second loop (14) at a first connection point (16) upstream of the filter (28) and the second loop (14) connects to the first loop (12) at a second connection point (18) downstream of the filter (28) such that the first loop (12) supplies the feed from the feed tank (22) to the second loop (14) at the first connection point (16) and the second loop (14) supplies retentate to the first loop (12) at the second connection point (18) from where a part of the retentate is returned to the feed tank (22),
wherein at least all wetted parts of the device assembly (10) are preferably single-use parts, and
wherein the inner diameter of the first tubing (20) is smaller than the inner diameter of the second tubing (32).

2. Device assembly (10) for performing a production-scale tangential flow filtration of a feed, preferably in a batch feed and bleed process, wherein the device assembly (10) comprises
a first loop (12) including a first tubing (20), a feed tank (22), and actuators and sensors adapted into the first tubing (20); and
a second loop (14) including second tubing (32) and a filter (28), preferably a hollow fiber filter;
wherein the first loop (12) connects to the second loop (14) at a first connection point (16) downstream of the filter (28) and the second loop (14) connects to the first loop (12) at a second connection point (18) upstream of the filter (28) such that the first loop (12) supplies the feed from the feed tank (22) to the second loop (14) at the second connection point (18) and the second loop (14) supplies retentate to the first loop (12) at the first connection point (18) from where a part of the retentate is returned to the feed tank (22),
wherein at least all wetted parts of the device assembly (10) are preferably single-use parts, and
wherein the inner diameter of the first tubing (20) is smaller than the inner diameter of the second tubing (32).

3. Device assembly (10) according to any of claims 1 and 2, wherein the second tubing (32) has a standard inner diameter of 2" (5.08 cm) or more.

4. Device assembly (10) according to any of claims 1 and 2, wherein the first tubing (20) has a standard inner diameter of 1" (2.54 cm) or less.

5. Device assembly (10) according to any of the preceding claims, wherein the second tubing (32) includes rigid piping, while the first tubing (20) preferably includes flexible hoses.

6. Device assembly (10) according to any of the preceding claims, wherein the device assembly (10) comprises control means configured to control the flow rate and the pressure in the first loop (12) and in the second loop (14), especially the transmembrane pressure across the filter (28).

7. Device assembly (10) according to claim 6, wherein the control means are configured such that in the whole second loop (14) the flow rate and the pressure are high, whereas in the first loop (12) the flow rate and the pressure in the tubing directly leading to and away from the feed tank (22) are low.

8. Device assembly (10) according to claim 7, wherein the control means include a feed pump (24) in the first loop (12) downstream of the feed tank (22) configured to supply the feed to the second loop (14) with a low flow rate and a high pressure, a first circulation pump (30), preferably a centrifugal pump, in the second loop (14) upstream of the filter (28), and a retentate control valve (26) in the first loop (12) upstream of the feed tank (22), the transmembrane pressure being mainly controlled jointly by the feed pump (24) and the retentate control valve (26).

9. Device assembly (10) according to claim 7, wherein the control means include a feed pump (24) in the first loop (12) downstream of the feed tank (22) configured to supply the feed to the second loop (14) with a low flow rate and a high pressure, a first circulation pump (30), preferably a centrifugal pump, in the second loop (14) upstream of the filter (28), and a bleed pump (44) in the first loop (12) upstream of the feed tank (22), the transmembrane pressure being mainly controlled jointly by the feed pump (24) and the bleed pump (44).

10. Device assembly (10) according to claim 6, wherein the control means are configured such that in the second loop (14) the flow rate and the pressure are high upstream and downstream of the filter (28), the flow rate is high and the pressure is low at the first and second connection points (16, 18), and in the whole first loop (12) the flow rate and the pressure are low.

11. Device assembly (10) according to claim 10, wherein the control means include a feed pump (24) in the first loop (12) downstream of the feed tank (22) configured to supply the feed to the second loop (14) with a low flow rate and a low pressure, a first centrifugal circulation pump (30) and a second centrifugal pump (46) in the second loop (14) upstream and downstream of the filter (28), respectively, the transmembrane pressure being mainly controlled jointly by the first and second centrifugal circulation pumps (30, 46), the first and second centrifugal circulation pumps (30, 46) being configured to establish a first flow direction over the filter (28) and an alternative second flow direction over the filter (28).

12. Device assembly (10) according to claim 7, wherein the control means are configured such that in the whole second loop (14) the flow rate and the pressure are high, whereas in the first loop (12) the flow rate and the pressure in the tubing directly leading to and away from the feed tank (22) are low.

13. Device assembly (10) according to claim 12, wherein the control means include a feed pump (24) in the first loop (12) downstream of the feed tank (22) configured to supply the feed to the second loop (14) with a low flow rate and a high pressure, a first circulation pump (30) and a first circulation on/off valve (50) connected in parallel upstream of the filter (28) in the second loop (14), a second circulation pump (48) and a second circulation on/off valve (52) connected in parallel downstream of the filter (28) in the second loop (14), and a retentate control valve (26) in the first loop (12) upstream of the feed tank (22), the transmembrane pressure being mainly controlled jointly by the feed pump (24) and the retentate control valve (26), the first and second circulation pumps (30, 48) and the first and second circulation on/off valves (50, 52) being configured to establish a first flow direction over the filter (28) and an alternative second flow direction over the filter (28).

14. Device assembly (10) according to claim 7, wherein the control means include a feed pump (24) in the first loop (12) downstream of the feed tank (22) configured to supply the feed to the second loop (14) with a low flow rate and a high pressure, a first circulation pump (30) in the second loop (14) upstream of the filter (28), one or more circulation on/off valves (54) in different lines of the second loop (14), and a retentate control valve (26) in the first loop (12) upstream of the feed tank (22), the transmembrane pressure being mainly controlled jointly by the feed pump (24) and the retentate control valve (26), the circulation on/off valves (54) being configured to establish a first flow path in which the first circulation pump (30) provides a first flow direction over the filter (28), and an alternative second flow path in which the circulation pump (30) provides a second flow direction over the filter (28).

15. Device assembly (10) according to any of the preceding claims, wherein the second loop (14) includes a plurality of filters (28) connected in parallel.

16. Device assembly (10) according to any of the preceding claims, wherein the second loop (14) includes plurality of filters (28) connected in series.

17. Device assembly (10) according to any of the preceding claims, further comprising a permeate pump (34), preferably a peristaltic pump, configured to control the flow rate and the pressure of the permeate.

18. Device assembly (10) according to any of the preceding claims, wherein the device assembly (10) further comprises a UV measurement or UV-Vis spectroscopy setup (42) in the permeate stream configured to detect breakthrough of the filter (28) and/or a UV measurement or UV-Vis spectroscopy setup (42) in the retentate stream configured to monitor an analyte.

19. Device assembly (10) according to any of the preceding claims, wherein the pumps and valves are controlled by a central process control unit or SCADA system.

20. Device assembly (10) according to any of the preceding claims, wherein the central process control unit or the SCADA system is configured to control the concentration gradient between the first loop (12) and the second loop (14), preferably by adjusting a ratio of feed stream to bleed stream being directed from and redirected to the feed tank (22), respectively.

21. Method of performing production-scale tangential flow filtration of a feed, preferably in a batch feed and bleed process, wherein the method makes use of the device assembly (10) according to any of the preceding claims and comprises the following steps:
- the first loop (12) supplying feed from the feed tank (22) to the second loop (14) at the first connection point (16), and
- the second loop (14) supplying retentate to the first loop (12) at the second connection point (18) from where a part of the retentate is returned to the feed tank (22); or
- the first loop (12) supplying feed from the feed tank (22) to the second loop (14) at the second connection point (18), and
- the second loop (14) supplying retentate to the first loop (12) at the first connection point (18) from where a part of the retentate is returned to the feed tank (22).
